(19) Europäisches Patentamt

European Patent Office

Office européen des brevets

(11) **EP 1 332 772 A2**

(12) **EUROPEAN PATENT APPLICATION**

(43) Date of publication:
06.08.2003 Bulletin 2003/32

(51) Int Cl.⁷: **A61P 17/00**, A61K 7/48

(21) Application number: 03250700.6

(22) Date of filing: 04.02.2003

(84) Designated Contracting States:
**AT BE BG CH CY CZ DE DK EE ES FI FR GB GR
HU IE IT LI LU MC NL PT SE SI SK TR**
Designated Extension States:
**AL LT LV MK RO**

(30) Priority: **05.02.2002 US 67596**

(71) Applicant: **INTERNATIONAL FLAVORS &
FRAGRANCES INC.
New York New York 10019 (US)**

(72) Inventors:
• **Flammer, Linda J.
Essex Country, New Jersey 07079 (US)**

• **Grainger, Brian T.
Somerset County, New Jersey 08540 (US)**
• **Boden, Richard M.
Monmouth County, New Jersey 07712 (US)**
• **Christensen, Carol
Middlesex County, New Jersey 08840 (US)**

(74) Representative: **Mercer, Christopher Paul et al
Carpmaels & Ransford
43, Bloomsbury Square
London WC1A 2RA (GB)**

(54) **Anti-dandruff and anti-itch compositions containing a cooling sensate material and a cooling sensate enhancer**

(57)    Described arc anti-dandruff and anti-itch compositions comprising:

(a) an antidandruff agent;
(b) a cooling sensate material; and
(c) a cooling sensate.

Also described are personal care products for reduction of itching including shampoos, soaps, ointments and creams which contain the anti-dandruff and anti-itch compositions.

**EP 1 332 772 A2**

**Description**

**FIELD OF THE INVENTION**

Our invention relates to anti-dandruff and anti-itch compositions comprising:

(a) an anti-dandruff agent, such as the zinc salt of 1-hydroxy-2-pyridine thione or an anti-itch agent such as beta-methasone valerate;
(b) a cooling sensate material, such as menthol or a mixture of menthol with 2-isopropyl-N,2,3-trimethylbutyramide; and
(c) a cooling sensate enhancer, such as vanillyl butyl ether, nonylic acid vanillamide or Jambu oleoresin.

Our invention also relates to anti-dandruff shampoos containing such anti-dandruff compositions which shampoos can optionally contain fragrance. Our invention further relates to a method for effecting significant reduction of itching of the scalp, medically known as *pruritis,* resulting from dandruff, by means of application to the scalp of such anti-dandruff shampoos. Our invention also relates to personal care products containing the aforementioned anti-itch compositions for reduction of itching including shampoos, soaps, ointments and creams.

**BACKGROUND OF THE INVENTION**

Seborrheic dermatitis, *pruritis* and dandruff represent wide-spread cosmetic problems. Methods for the treatment of seborrheic dermatitis, *pruritis* and dandruff are known in the art. Among the most common treatment regimens are washing with shampoos containing chloroxylenol, pyridinethione heavy metal salts including zinc, cadmium, magnesium, tin, aluminum and zirconium; salts such as zinc pyrithione, sulfur, selenium sulfide, salicylic acid, piroctone olamine also known as octopirox; hexachlorophene, resorcinol, coal tar, coal tar extracts, coal tar solutions, ketoconazole, alkali metal salts and ammonium salts of low molecular weight huminates with a mean molecular weight of 1000 with a range of 300 to 1500, and certain cationics such as cetyldimethylbenzylammonium bromide followed by rinsing, as disclosed in U.S. Patent Numbers 4,470,982, 5,494,675, 5,641,480, 5,730,965 and 6,294,186, each of which is incorporated herein by reference. Shampoos which contain cooling sensates such as menthol and menthyl lactate are disclosed in U.S. Patent No. 6,294,186, cited above.

However, the uses of the aforementioned regimens have the disadvantage of stimulating the treated areas of the mammalian epidermis including the scalp to "grease up" within a relatively short period of time subsequent to use of the treatment agent, particularly when zinc pyrithione is employed in the personal care treatment agent, even when it is used in combination with cooling sensates such as menthol. Furthermore, certain sensory effects, such as: substantial soothing effects on use and post-use, substantial deep-cleansed effects on use and post-use, post-use significant itch reduction, substantial tingling effects on use and post-use, substantial warming effects on use and post-use, substantial cooling effects on use and post-use and a significant enhanced menthol/medicated aroma on use and post-use have heretofore not been achieved by the regimens disclosed in the prior art.

Accordingly, it is an object of our invention to provide compositions, personal care products containing same, and methods of employing such compositions and personal care products for the treatment of seborrheic dermatitis, *pruritis* and dandruff which (1) substantially avoid the aforementioned disadvantages and (2) provide the sensory effects.

Although the use in shampoos and other personal care products, e.g., lotions and creams, in general, of cooling sensates in combination with cooling sensate enhancers, are disclosed in the prior art, published European Patent Application 1,121,927 A2 and U.S. Patent No. 6,328,982, herein incorporated by reference, nothing in the prior art discloses or suggests, the unexpected and unobvious advantages obtained as a result of using cooling sensates such as menthol in combination with cooling sensate enhancers with anti-itch materials or anti-dandruff substances. The combination of these elements in a personal care product provide the following benefits for a substantial period of time post-use:

(i) a substantial soothing effect;
(ii) a deep-cleansed effect;
(iii) a significant itch reduction;
(iv) a substantial tingling effect;
(v) a substantial warming effect;
(vi) a substantial cooling effect; and/or
(vii) a significantly enhanced "menthol/medicated" aroma.

## SUMMARY OF THE INVENTION

Our invention provides anti-dandruff compositions and an anti-itch compositions comprising:

(a) an anti-dandruff agent or an anti-itch agent;
(b) a cooling sensate material; and
(c) a cooling sensate enhancer material.

The anti-dandruff compositions of our invention are useful as key components in anti-dandruff shampoos; and the anti-itch compositions of our invention are useful as key components in personal care products, specifically shampoos, ointments and creams. The shampoos and personal care products of our invention can optionally contain fragrances. On application and for an extended period of time subsequent to application to the mammalian epidermis of the shampoos and personal care products of our invention, at least one of the following effects is exerted:

(i) a substantial soothing effect;
(ii) a deep-cleansed effect;
(iii) a significant itch reduction;
(iv) a substantial tingling effect;
(v) a substantial warming effect;
(vi) a substantial cooling effect; or
(vii) a significantly enhanced "menthol/medicated" aroma in a magnitude substantially and significantly greater than if the cooling sensate enhancer were not included in the anti-dandruff or anti-itch composition.

## DETAILED DESCRIPTION OF THE INVENTION

In the anti-dandruff composition of our invention, the weight ratio of anti-dandruff agent:cooling sensate material:cooling sensate enhancer material is in the range of from about 0.7 to about 1.5 antidandruff agent: from about 0.5 to about 1.5 cooling sensate material: and from about 0.001 to about 0.1 cooling sensate enhancer. More preferably, the weight ratio of cooling sensate material:cooling sensate enhancer material is from about 1:0.1 to about 1:0.01.

In the anti-itch composition of our invention, the weight ratio of anti-itch agent:cooling sensate material:cooling sensate enhancer material is in the rage of from about 0.7 up to about 1.5 anti-itch agent:from about 0.5 up to about 1.5 cooling sensate material:and from about 0.001 up to about 0.1 cooling sensate enhancer material. More preferably, the weight ratio of cooling sensate material:cooling sensate enhancer material is from about 1:0.1 down to about 1:0.01.

Examples of anti-dandruff agents useful in the practice of our invention are separately or in combination: chloroxylenol, as disclosed in U.S. Patent No. 5,730,965, the disclosure of which is incorporated herein by reference, pyridinethione heavy metal salts, e.g., zinc, cadmium, magnesium, tin, aluminum and zirconium salts, such as, zinc pyrithione, as disclosed in U.S. Patent No. 4,470,982, the disclosure of which is incorporated herein by reference, 1-hydroxy pyridones as disclosed in U.S. Patent No. 4,185,106, the disclosure of which is herein incorporated by reference, azole antimycotics, as disclosed in U.S. Patent No. 4,472,421, the disclosure of which is incorporated herein by reference, sulfur, ketoconazole, selenium sulfide, salicylic acid, piroctone olamine, hexachlorophene, resorcinol, coal tar, coal tar extracts, coal tar solutions, alkali metal salts and ammonium salts of low molecular weight huminates with a mean molecular weight of 1000 with a range of 300 to 1500, as disclosed in U.S. Patent No. 5,494,675, the disclosure of which is incorporated herein by reference, and certain cationics such as cetyldimethylbenzylammonium bromide. The anti-dandruff agent is provided in an amount sufficient to prevent or reduce dandruff, the weight percent of the anti-dandruff agent found in the shampoo is typically from about 0.1% to about 3.0%.

Examples of anti-itch agents useful in the practice of our invention are separately or in combination: chamomile, eucalyptus, camphor, talc, hydrocortisone, betamethasone valerate, fluocinolone acetonide, hydrocortisone valerate, triamcinolone acetonide, betamethasone dipropionate, halcinonide, clobetasol propionate and halobetasol propionate. The weight percent of anti-itch agent found in a cream, soap is from about 0.02% to about 3.0%.

Examples of cooling sensate materials useful in the practice of our invention are: hydroxy-lower alkyl derivatives of para-menthane, for example, 2-hydroxymethyl menthol as described in U.S. Patent Number 4,029,759, the disclosure of which is incorporated herein by reference, 1-isopulegol, mint oil, spearmint oil, peppermint oil, methyl salicylate, menthone, menthone glyceryl ketal, menthol, p-menthane diol, menthyl lactate, mono-menthyl succinate, alkali metal salts of mono-menthyl succinate, alkaline earth metal salts of mono-menthyl succinate, mono-menthyl glutarate, alkali metal salts of mono-menthyl glutarate, menthoxy-$C_1$-$C_5$ alkanols for example (d,1)-2-(5'-methyl-2'-(methylethyl)cyclohexyloxy)-ethan-1-ol, as disclosed in published European Patent Application 1,122,233 A1, menthoxy $C_1$-$C_5$ alkyl ethers, $C_1$-$C_3$ alkyl or dialkyl-N-substituted menthane carboxamides, including N-ethyl-p-menthane carboxamide, as disclosed in U.S. Patent Numbers 4,296,093 and 6,303,817, the disclosures of which are incorporated herein by reference; menthoxy-$C_1$-$C_5$ alkanediols, $C_1$-$C_3$ alkyl or dialkyl-N-substituted $C_5$-$C_{12}$ alkyl carboxamides, for example, N,2,3-trimethyl-2-ethylbutanamide and N,2,3-trimethyl-2-isopropyl-butyramide, as disclosed in U.S. Patent Number 4,153,679, the disclosure of which is incorporated herein by reference; alkylcyclohexyl sulfones and sulfoxides, for example, n-hexyl-1,2-diethylcyclohexyl sulfoxide as disclosed in U.S. Patent Number 4,032,661, the disclosure of which is incorporated herein by reference; and cyclic α-keto enamines, for example, 3-methyl-2-(1-pyrrolidinyl)-2-cyclohexen-1-one (as disclosed in Ottinger et al. *J.Agric.Food Chem.* 2001, 49, 5383-5390.

Examples of cooling sensate enhancer materials which are sometimes referred to as warming sensates include: vanillyl $C_2$-$C_8$ alkyl ethers, such as vanillyl-n-butyl ether, menthoxymethyl dihydroxyphenyl dioxolanes and menthoxymethyl hydroxymethoxyphenyl dioxolanes, for example, 4-(1-menthoxymethyl)-2-phenyl-1,3-dioxolane, as disclosed in U.S. Patent 5,545,424 the disclosure of which is herein incorporated by reference, $C_7$-$C_{12}$ alkanoic acid vanillamides, for example, nonylic acid vanillamide, vanillin or ethyl vanillin $C_3$-$C_6$ alkylene glycol acetals, ginger oleoresin, capsicum oleoresin and capsaicin.

Examples of cooling sensate materials which are sometimes referred to as tingling sensates include: Jambu oleoresin, Spilanthol, saanshool-I, saanshool-II, sanshoamide, *Piper nigrum, Zanthoxylum peperitum,* chavicine and piperine.

The cooling sensate material and cooling sensate enhancer are provided in an amount sufficient to provide the beneficial effects described herein. These effects include but are not limited to a substantial soothing effect; a deep-cleansed effect; itch reduction; tingling effect; warming effect; cooling effect; and/or menthol aroma.

The anti-dandruff and anti-itch personal care compositions of our invention may optionally contain a fragrance, each of the components of which has a C $\log_{10}$P (i) in the range of from about 1 up to about 3, without restriction on the molecular weight of each of said components, (ii) in the range of from about greater than 3 up to about 10 for components each of which has a molecular weight in the range of from about 120 up to about 350 or (iii) in the range of from about 1 up to about 3, without restriction on the molecular weight of each of said components and in the range of from about greater than 3 up to about 10 for components each of which has a molecular weight in the range of from about 120 up to about 350, wherein P is the n-octanol/water partition coefficient of the fragrance component. The concentration range of the fragrance in the personal care compositions of our invention is preferably from about 0.03% up to about 5.0% by weight of the personal care composition.

The $\log_{10}$P of many perfume ingredients has been reported; for example, the Pomona92 database, available from Daylight Chemical Information Systems, Inc., Daylight CIS, Irvine, California, contains many, along with citations to the original literature. However, the $\log_{10}$P value are most conveniently calculated by the "CLOGP" program, also available from Daylight CIS. This program also lists experimental $\log_{10}$P values when they are available in the Pomona92 database. The "calculated $\log_{10}$P" (C$\log_{10}$P) is determined by the fragment approach of Hansch and Leo cf., A. Leo in Comprehensive Medicinal Chemistry, Vol.4, C. Hansch, P.G. Sammens, J. B. Taylor and C.A. Ramsden, Eds., p.295, Pergamon Press, 1990. The fragment approach is based on the chemical structure of each perfume ingredient, and takes into account the numbers and types of atoms, the atom connectivity and the chemical bonding. The C$\log_{10}$P value which are the most reliable and widely used estimates for this physicochemical property, are preferably used instead of the experimental $\log_{10}$P values for the selection of perfume ingredients which are useful in the anti-dandruff shampoos and anti-itch personal care products of our invention.

Specific examples of preferred fragrance components useful in the personal care products of our invention, and the molecular weights and C$\log_{10}$P values of each of said components for each of groups (i), (ii) and (iii) defined supra are as follows:

Fragrance Component Group (i):

| Fragrance Component | Clog$_{10}$P value | Molecular Weight |
|---|---|---|
| Benzaldehyde | 1.480 | 106.12 |
| benzyl acetate | 1.960 | 150.17 |
| laevo-carvone | 2.083 | 150.22 |
| Geraniol | 2.649 | 154.26 |
| cis-jasmone | 2.712 | 164.25 |
| β-phenylethyl alcohol | 1.183 | 122.17 |
| α-terpineol | 2.569 | 154.25 |

(continued)

| Fragrance Component | Clog$_{10}$P value | Molecular Weight |
|---|---|---|
| δ-nonalactone | 2.760 | 156.23 |
| Nerol | 2.649 | 154.25 |
| iso-eugenol | 2.547 | 164.21 |
| Eugenol | 2.307 | 164.21 |

Fragrance Component Group (ii):

| Fragrance Component | Clog$_{10}$P value | Molecular Weight |
|---|---|---|
| amyl salicylate | 4.601 | 208.26 |
| benzyl salicylate | 4.383 | 228.25 |
| β-caryophyllene | 6.333 | 204.36 |
| Cedrol | 4.530 | 222.37 |
| cedryl acetate | 5.436 | 264.41 |
| cedryl formate | 5.070 | 238.37 |
| cyclohexyl salicylate | 5.265 | 220.29 |
| γ-dodecalactone | 4.359 | 198.31 |
| ethyl undecylenate | 4.888 | 212.34 |
| geranyl anthranilate | 4.216 | 273.38 |
| α-irone | 3.820 | 206.33 |
| phenyl ethyl benzoate | 4.058 | 226.28 |
| phenylethyl phenyl acetate | 3.767 | 240.31 |
| 5-acetyl-1,1,2,3,3,6-hexamethyl indane | 5.977 | 258.41 |
| Cyclopentadecanolide | 6.246 | 240.39 |
| d-limonene | 4.232 | 136.24 |
| cis-p-t-butylcyclohexyl acetate | 4.019 | 198.31 |
| amyl cinnamic aldehyde | 4.324 | 202.30 |
| linalyl benzoate | 5.233 | 258.36 |

Fragrance Component Group (iii):

| Fragrance Component | Clog$_{10}$P value | Molecular Weight |
|---|---|---|
| benzaldehyde | 1.480 | 106.12 |
| benzyl acetate | 1.960 | 150.17 |
| laevo-carvone | 2.083 | 150.22 |
| geraniol | 2.649 | 154.26 |
| cis-jasmone | 2.712 | 164.25 |
| β-phenylethyl alcohol | 1.183 | 122.17 |

(continued)

| Fragrance Component | Clog$_{10}$P value | Molecular Weight |
|---|---|---|
| α-terpineol | 2.569 | 154.25 |
| δ-nonalactone | 2.760 | 156.23 |
| dihydromyrcenol | 3.03 | 156.27 |
| δ-undecalactone | 3.830 | 184.28 |
| amyl cinnamate | 3.771 | 218.30 |
| benzophenone | 3.120 | 182.22 |
| α-irone | 3.820 | 206.33 |
| nerol | 2.649 | 154.25 |
| 2-methoxynaphthalene | 3.235 | 158.20 |
| musk ketone | 3.014 | 294.30 |
| musk tibetine | 3.831 | 266.30 |
| myristicin | 3.200 | 192.22 |
| 6-phenyl heptanol-2 | 3.478 | 193.30 |
| 1-phenyl hexanol-5 | 3.299 | 178.28 |
| α-santalol | 3.800 | 220.36 |
| iso-eugenol | 2.547 | 164.21 |
| linalyl acetate | 3.500 | 196.29 |
| eugenol | 2.307 | 164.21 |
| amyl salicylate | 4.601 | 208.26 |
| benzyl salicylate | 4.383 | 228.25 |
| β-caryophyllene | 6.333 | 204.36 |
| cedrol | 4.530 | 222.37 |
| cedryl acetate | 5.436 | 264.41 |
| cedryl formate | 5.070 | 238.37 |
| cyclohexyl salicylate | 5.265 | 220.29 |
| γ-dodecalactone | 4.359 | 198.31 |
| ethyl undecylenate | 4.888 | 212.34 |
| geranyl anthranilate | 4.216 | 273.38 |
| β-phenylethyl benzoate | 4.058 | 226.38 |
| β-phenylethyl phenyl acetate | 3.767 | 240.31 |
| 5-acetyl-1,1,2,3,3,6-hexamethyl indane | 5.977 | 258.41 |
| cyclopentadecanolide | 6.246 | 240.39 |
| d-limonene | 4.232 | 136.24 |
| cis-p-t-butylcyclohexyl acetate | 4.019 | 198.31 |
| amyl cinnamic aldehyde | 4.324 | 202.30 |
| linalyl benzoate | 5.233 | 258.36 |

As stated herein, in the practice of our invention, the personal care products of our invention as well as for a

EP 1 332 772 A2

substantial period of time post-use, e.g. from 1-30 minutes post-use exhibit:

  i. a substantial soothing effect;
  ii. a deep-cleansed effect;
  iii. a significant itch reduction;
  iv. a substantial tingling effect;
  v. a substantial warming effect;
  vi. a substantial cooling effect; and/or
  vii. a significantly enhanced "menthol/medicinal" aroma.

The term, "soothing effect" is intended herein to mean an effect measured using a combination of the following "soothing components":

  A. irritation reduction with respect to the scalp, neck and facial areas of the mammalian epidermis;
  B. tightness reduction of the scalp, neck and facial areas of the mammalian epidermis;
  C. dryness feeling reduction of the scalp, neck and facial areas of the mammalian epidermis;
  D. moisturized feeling of the scalp, neck and facial areas of the mammalian epidermis; and/or
  E. coated or protected feeling of the scalp, neck and facial areas of the mammalian epidermis.

This soothing effect is herein, including in the Examples set forth below are quantified according to a scale of 1-10 with 10 being the most desirable soothing effect and 1 signifying no soothing effect. The soothing effect scaled value, $V_S$ may be calculated using scaled value terms for each of the aforementioned soothing components, A, B, C, D and E, supra, in accordance with the following algorithm:

$$b_1\alpha + b_2\beta + b_3\gamma + b_4\delta + b_5\varepsilon = V_S$$

wherein the scaled values of the soothing components, A, B, C, D and E are, respectively, $\alpha$, $\beta$, $\gamma$, $\delta$ and $\varepsilon$ and $b_1$, $b_2$, $b_3$, $b_4$ and $b_5$ are each, respectively, the magnitude of importance of each of the "soothing components", A, B, C, D and E, with the provisos that:

$$\Sigma b_i = 1, \text{ and } 0.1 \leq b_i \leq 0.5$$

The value of each of the terms, $b_i$ depend on the desired magnitude of personal and washing variables, e.g., type of hair, time of washing, and number of wash-rinse cycles. In the Examples contained herein, infra, where the value of $V_S$ is shown, each of the terms $b_i$ is 0.2.
The term, "deep-cleansed effect as measured by the IFF 'DC' test" is intended herein to mean an effect measured using a combination of the following "deep cleansing components":

  V.   audibility of a 'squeak' sound when a swatch of hair held between the thumb and forefinger is pulled through the fingers. The greater the number of decibels produced, the higher the value of the audibility of 'squeak' sound effect;
  W.   degree of smoothness;
  X.   degree of silkiness;
  Y.   degree of grease-free and oil-free slipperiness; and/or
  Z.   Degree of vibration and roughness.

The "deep-cleansed" effect is herein (including in the Examples set forth infra) quantified according to a scale of "1" to "10" with a value of "10" being the most desirable "deep-cleansed" effect and a value of "1" signifying no "deep-cleansed" effect with an oily, greasy feel being present. The "deep-cleansed" effect scaled value, $V_{DC}$ may be calculated using scaled value terms for each of the aforementioned "deep cleansing components", V, W, X, Y and Z, supra, in accordance with the following algorithm:

$$a_1\chi + a_2\phi + a_3\eta + a_4\kappa + a_5\lambda = V_{DC}$$

wherein the scaled values of the "deep cleansing components", V, W, X, Y and Z are, respectively, $\chi$, $\phi$, $\eta$, $\kappa$ and $\lambda$ and

7

$a_1$, $a_2$, $a_3$, $a_4$ and $a_5$ are each, respectively, the magnitude of importance of each of the "deep cleansing components", V, W, X, Y and Z with the provisos that:

$$\Sigma a_i = 1, \text{ and } 0.1 \leq a_i \leq 0.5$$

The value of each of the terms, $a_i$ depend on the desired magnitude of personal and washing variables, e.g., type of hair, time of washing and number of wash-rinse cycles. In the Examples contained herein, infra, where the value of $V_{DC}$ is shown, each of the terms $a_i$ is 0.2.

The term itching is herein intended to mean the peculiar and unpleasant irritating sensation of a given portion of the epidermis, e.g., scalp, face and neck of a person, that provokes the desire of said person to scratch said given portion of the epidermis.

The term tingling is herein intended to mean a lively "pins-and-needles" sensation of a given portion of the epidermis, e.g., scalp, face and neck, of a person.

The term "warming" is herein intended to mean the degree to which a product produces the sensation of heat, when applied to a portion of the epidermis, e.g., scalp. face and neck, of a person.

The term "cooling" is herein intended to mean the degree to which a product imparts a cool feeling when applied to a portion of the epidermis, e.g., scalp, face and neck of a person.

Each of the effects: "significant itch reduction", "substantial tingling", "substantial warming" and "substantial cooling" is measured with reference to the scalp, facial and neck portions of the mammalian epidermis; and each of these effects are herein, including in the Examples, infra also quantified according to a scale of 1-10 with a value of 1 signifying the most desirable effect, and a value of 1 signifying no such effect.

The significantly enhanced menthol/medicated aroma effect is herein measured with reference to the eyes and nasal pathways on use of the personal products of our invention; and such effect is herein including in the Examples, also quantified according to a scale of 1-10 with a value of 10 signifying the most desirable effect and a value of 1 signifying no such effect.

The personal care products useful in the practice of our invention include shampoos, creams, ointments and soaps. More specifically shampoos as exemplified in U.S. Patent Numbers 4,470,982, 5,624,666, 5,641,480, 5,955,066, 6,200,554, 6,248,315 and 6,297,203, the disclosures of each patent are incorporated by reference herein, are useful in the practice of our invention. In addition to shampoos, the personal care products useful in the practice of the invention include soaps, ointments, creams and lotions, all of which are well known in the art. These forms and methods for making them are disclosed in US Patent Numbers 6,338,855, 6,210,695 and 6,299,900 the contents of which are incorporated by reference.

The following non-limiting examples are presented for purposes of illustration, unless noted to the contrary all ingredients are provided by weight.

## EXAMPLE A

## PREPARATION OF FRAGRANCE FOR USE IN SHAMPOO OF EXAMPLE B

The following fragrance is prepared for use with the shampoo of Example B, infra:

| Ingredients | Parts by Weight |
| --- | --- |
| α-irone | 7.0 |
| myristicin | 4.0 |
| 2-methoxynaphthalene | 3.0 |
| benzaldehyde | 2.0 |
| β-phenylethyl alcohol | 12.0 |
| nerol | 7.0 |
| eugenol | 8.0 |
| isoeugenol | 2.0 |
| amyl salicylate | 4.0 |
| β-caryophyllene | 14.0 |

(continued)

| Ingredients | Parts by Weight |
|---|---|
| cedryl acetate | 16.0 |
| cyclohexyl salicylate | 4.0 |
| γ-dodecalactone | 3.0 |
| geranyl anthranilate | 3.0 |

## EXAMPLE B

### PREPARATION OF FRAGRANCE-CONTAINING SHAMPOO BASE FOR USE IN CONJUNCTION WITH EXAMPLES I-VII, INCLUSIVE, INFRA

At the rate of 0.8%, the fragrance prepared according to Example A, is admixed with the following aqueous shampoo base:

| Component | Parts by Weight |
|---|---|
| ammonium lauryl sulfate (27% aqueous solution) | 56.0 |
| citric acid | 0.50 |
| sodium citrate | 0.50 |
| coconut monoethanolamide | 5.0 |
| ethylene glycol distearate | 3.0 |
| methyl paraben | 0.50 |
| propyl paraben | 0.50 |
| color solution | 0.20 |
| Water | 33.8 |

## EXAMPLE I

To the shampoo of Example B, zinc pyrithione at the rate of 1% and menthol at the rate of 0.5% is added. The effects exerted during use and post-use of the resulting shampoo are set forth in the Tables of Results.

## EXAMPLE II

To the shampoo of Example B, zinc pyrithione at the rate of 1%, menthol at the rate of 0.5% and N,2,3-trimethyl-2-isopropyl-butyramide at the rate of 0.35% is added. The effects exerted during use and post-use of the resulting shampoo are set forth in the Tables of Results.

## EXAMPLE III

To the shampoo of Example B, zinc pyrithione at the rate of 1%, menthol at the rate of 0.5%, N,2,3-trimethyl-2-isopropyl-butyramide at the rate of 0.35% and 0.02% Jambu oleoresin is added. The effects exerted during use and post-use of the resulting shampoo are set forth in the Tables of Results.

## EXAMPLE IV

To the shampoo of Example B, zinc pyrithione at the rate of 1%, menthol at the rate of 0.5%, N,2,3-trimethyl-2-isopropyl-butyramide at the rate of 0.35% and vanillyl-n-butyl ether at the rate of 0.05% is added. The effects exerted during use and post-use of the resulting shampoo are set forth in the Tables of Results.

## EXAMPLE V

To the shampoo of Example B, zinc pyrithione at the rate of 1%, menthol at the rate of 0.5%, N,2,3-trimethyl-2-isopropyl-butyramide and nonylic acid vanillamide at the rate of 0.002% is added. The effects exerted during use and post-use of the resulting shampoo are set forth in the Tables of Results.

## EXAMPLE VI

To the shampoo of Example B supra, zinc pyrithione at the rate of 1%, menthol at the rate of 0.5% and Jambu oleoresin at the rate of 0.2% is added. The effects exerted during use and post-use of the resulting shampoo are set forth in the Tables of Results.

## EXAMPLE VII

To the shampoo of Example B supra, zinc pyrithione at the rate of 1% and Jambu oleoresin at the rate of 0.2% is added. The effects exerted during use and post-use of the resulting shampoo are set forth in the Tables of Results.

## TABLES OF RESULTS

In the following Tables I, II and III, the 'effects' for which values, determined using a 17 member panel; and as defined supra, including $V_S$ and $V_{DC}$ are set forth are as follows:

    i. a substantial soothing effect;
    ii. a deep-cleansed effect;
    iii. a significant itch reduction;
    iv. a substantial tingling effect;
    v. a substantial warming effect;
    vi. a substantial cooling effect; and/or
    vii a significantly enhanced "menthol/medicinal" aroma.

| TABLE I OF RESULTS | | | | | | |
|---|---|---|---|---|---|---|
| Example | Value of Effect(i) ($V_S$) (soothing) | | Value of Effect (ii) ($V_{DC}$)(deep cleansing) | | Value of Effect(iii) (itch reduction) | |
|  | on use | 5 min. post-use | on use | 5 min. post-use | on use | 5 min. post-use |
| I | 6 | 5 | 8 | 8 | 7 | 7 |
| II | 10 | 10 | 7 | 7 | 10 | 7 |
| III | 10 | 10 | 7 | 6 | 10 | 10 |
| IV | 6 | 5 | 7 | 6 | 10 | 3 |
| V | 10 | 10 | 7 | 6 | 10 | 10 |
| VI | 6 | 5 | 10 | 10 | 10 | 1 |
| VII | 3 | 3 | 3 | 3 | 10 | 3 |
| B | 3 | 3 | 3 | 3 | 7 | 7 |

| TABLE II OF RESULTS | | | | | | |
|---|---|---|---|---|---|---|
| Example | Value of Effect(iv) (tingling) | | Value of Effect (v) (warming) | | Value of Effect(vi) (cooling) | |
|  | on use | 5 min.post-use | on use | 5 min. post-use | on use | 5 min. post-use |
| I | 8 | 8 | 9 | 6 | 6 | 6 |
| II | 9 | 10 | 9 | 6 | 9 | 7 |

(continued)

**TABLE II OF RESULTS**

| Example | Value of Effect(iv) (tingling) | | Value of Effect (v) (warming) | | Value of Effect(vi) (cooling) | |
|---|---|---|---|---|---|---|
| | on use | 5 min.post-use | on use | 5 min. post-use | on use | 5 min. post-use |
| III | 8 | 7 | 9 | 6 | 7 | 6 |
| IV | 9 | 8 | 10 | 8 | 7 | 6 |
| V | 10 | 9 | 10 | 6 | 9 | 8 |
| VI | 10 | 7 | 9 | 5 | 9 | 4 |
| VII | 3 | 5 | 9 | 5 | 4 | 3 |
| B | 1 | 1 | 8 | 7 | 3 | 1 |

**TABLE III OF RESULTS**

| Example | Value of Effect(vii) (menthol/medicated) | |
|---|---|---|
| | on use | 5 min. post-use |
| I | 8 | 8 |
| II | 10 | 10 |
| III | 8 | 8 |
| IV | 10 | 10 |
| V | 8 | 8 |
| VI | 5 | 5 |
| VII | 1 | 1 |
| B | 1 | 1 |

The foregoing Tables I, II and III of results are indicative of the unexpected, unobvious and advantages properties of the sensate compositions and personal care products defined of the invention.

From the foregoing, it will be appreciated that, although specific embodiments of the invention have been described herein for purposes of illustration, various modifications may be made without deviating from the spirit and scope of the invention. Accordingly, the invention is not limited except as by the appended claims.

**Claims**

1. An anti-dandruff composition comprising:

    (a) an anti-dandruff agent;
    (b) a cooling sensate material; and
    (c) a cooling sensate enhancer material.

2. The anti-dandruff composition of claim 1 wherein the cooling sensate enhancer material is selected from the group consisting of vanillyl $C_2$-$C_8$ alkyl ether, a menthoxymethyl dihydroxyphenyl dioxolane, a menthoxymethyl hydroxymethoxyphenyl dioxolane, a $C_7$-$C_{12}$ alkanoic acid vanillamide, a vanillin or ethyl vanillin $C_3$-$C_6$ alkylene glycol acetal, ginger oleoresin, capsicum oleoresin, and capsaicin.

3. The anti-dandruff composition of claim 1 wherein the cooling sensate material is selected from the group consisting of a hydroxy-lower alkyl derivative of para-menthane, 1-isopulegol, mint oil, spearmint oil, peppermint oil, methyl salicylate, menthone, menthone glyceryl ketal, menthol, p-menthane diol, menthyl lactate, mono-menthyl succinate, an alkali metal salt of mono-menthyl succinate, an alkaline earth metal salt of mono-menthyl succinate, mono-menthyl glutarate, an alkali metal salt of mono-menthyl glutarate, a menthoxy-$C_1$-$C_5$ alkanol, a menthoxy $C_1$-$C_5$ alkyl ether, a $C_1$-$C_3$ alkyl or dialkyl-N-substituted menthane carboxamide, a menthoxy-$C_1$-$C_5$ alkanediol, a $C_1$-$C_3$ alkyl or dialkyl-N-substituted $C_5$-$C_{12}$ alkyl carboxamide, an alkyl cyclohexyl sulfone, an alkyl cyclohexyl sulfoxide and a cyclic $\alpha$-keto enamine.

4. The anti-dandruff composition of claim 2 wherein the cooling sensate enhancer is selected from the group consisting of a vanillyl $C_2$-$C_8$ alkyl ether, a menthoxymethyl dihydroxyphenyl dioxolane, a menthoxymethyl hydroxymethoxyphenyl dioxolane, a $C_7$-$C_{12}$ alkanoic acid vanillamide, a vanillin or ethyl vanillin $C_3$-$C_6$ alkylene glycol acetal, ginger oleoresin, capsicum oleoresin, capsaicin, Jambu oleoresin, Spilanthol, saanshool-I, saanshool-II, sanshoamide, *Piper nigrum, Zanthoxylum peperitum,* chavicine and piperine.

5. The anti-dandruff composition of claim 2 wherein the cooling sensate enhancer is selected from the group consisting of Jambu oleoresin, Spilanthol, saanshool-I, saanshool-II, sanshoamide, *Piper nigrum, Zanthoxylum peperitum*, chavicine and piperine.

6. The anti-dandruff composition of claim 1 wherein the weight ratio of anti-dandruff agent: cooling sensate material : cooling sensate enhancer material is in the range of from 0.7 to 1.5 antidandruff agent : from 0.5 to 1.5 cooling sensate material : from 0.001 up to 0.1 cooling sensate enhancer.

7. The anti-dandruff composition of claim 6 wherein the weight ratio of cooling sensate material : cooling sensate enhancer material is from 1:0.1 to 1:0.01.

8. The anti-dandruff composition of claim 1 wherein the anti-dandruff agent is the zinc salt of 1-hydroxy-2-pyridinethione; the cooling sensate material comprises menthol; and the cooling sensate enhancer comprises n-nonylic acid vanillamide.

9. The antidandruff composition of claim 1 wherein the cooling sensate material is a mixture of menthol and 2-isopropyl-N,2,3-trimethylbutyramide and the cooling sensate enhancer is vanillyl-n-butyl ether.

10. The anti-dandruff composition of claim 1 wherein the cooling sensate material comprises menthol and the cooling sensate enhancer is Jambu oleoresin.

11. The anti-dandruff composition of claim 10 wherein the cooling sensate material is a mixture of menthol and 2-isopropyl-N,2,3-trimethylbutyramide.

12. An anti-dandruff shampoo comprising water, a shampoo base and the anti-dandruff composition of claim 1.

13. The anti-dandruff shampoo of claim 12 wherein the weight percent of anti-dandruff composition is from 0.5% to 2.5% by weight of the shampoo.

14. The anti-dandruff shampoo of claim 12 additionally comprising a fragrance, each of the components of which has a C $\log_{10}$P (i) in the range of from 1 up to 3, without restriction on the molecular weight of each of said components, (ii) in the range of from greater than 3 up to 10 for components each of which has a molecular weight in the range of from 120 up to 350 or (iii) in the range of from 1 up to 3, without restriction on the molecular weight of each of said components and in the range of from greater than 3 up to 10 for components each of which has a molecular weight in the range of from 120 up to 350, wherein **P** is the n-octanol/water partition coefficient of the fragrance component.

15. The shampoo of any one of claims 12 to 14 which exerts on the mammalian scalp at least one of:

    i. a substantial soothing effect;
    ii. a deep-cleansed effect as measured by the IFF squeak test;
    iii. a significant itch reduction;
    iv. a substantial tingling effect;
    v. a substantial warming effect;
    vi. a substantial cooling effect; or
    vii. a significantly enhanced "menthol/medicinal" aroma.

16. The shampoo of any one of claims 12 to 15 for use in reducing *pruritis* of the mammalian scalp caused by *seborrheic dermatitis.*

17. An composition for reducing an itch sensation occurring on the outer surface of the mammalian epidermis comprising:

   (a) an anti-itch agent;
   (b) a cooling sensate material; and
   (c) a cooling sensate enhancer.

18. The anti-itch composition of claim 17 wherein the cooling sensate enhancer material is selected from the group consisting of a vanillyl $C_2$-$C_8$ alkyl ether, a menthoxymethyl dihydroxyphenyl dioxolane, a menthoxymethyl hydroxymethoxyphenyl dioxolane, a $C_7$-$C_{12}$ alkanoic acid vanillamide, a vanillin or ethyl vanillin $C_3$-$C_6$ alkylene glycol acetal, ginger oleoresin, capsicum oleoresin, capsaicin, Jambu oleoresin, Spilanthol, saanshool-I, saanshool-II, sanshoamide, *Piper nigrum, Zanthoxylum peperitum,* chavicine and piperine.

19. The anti-itch composition of claim 18 wherein the cooling sensate material is N-ethyl-p-menthane-3-carboxamide.

20. The anti-dandruff composition of claim 3 wherein the cooling sensate material is N-ethyl-p-menthane-3-carboxamide.

21. A personal care composition comprising a personal care composition base and admixed therewith the anti-itch composition of claim 17.

22. The personal care composition of claim 21 wherein the personal care composition base is selected from the group consisting of a shampoo base, an ointment base, a soap base and a cream base.

23. The personal care composition of claim 21 or claim 22 for reducing an itch sensation occurring on the outer surface of the mammalian epidermis.